Europäisches Patentamt

European Patent Office

Office européen des brevets

(11) Publication number: 0 384 294
A2

# EUROPEAN PATENT APPLICATION

(21) Application number: 90102939.7

(22) Date of filing: 15.02.90

(51) Int. Cl.⁵: C12N 15/34, A01N 63/00, C07K 15/04

(30) Priority: 21.02.89 US 313226

(43) Date of publication of application:
29.08.90 Bulletin 90/35

(84) Designated Contracting States:
DE FR GB NL

(71) Applicant: BOYCE THOMPSON INSTITUTE FOR
PLANT RESEARCH, INC.
Tower Road
Ithaca, NY 14853(US)

(72) Inventor: Granados, Robert R.
5 Eagleshead Road
Ithaca, New York 14859(US)
Inventor: Hashimoto, Yoshifumi, Dept. of
Applied Biology
Kyoto Inst. of Technology, Matsugasaki
Sakyo-ku, Kyoto, 606(JP)

(74) Representative: Buchner, Otto, Dr. et al
Patentanwälte Dipl.-Ing. Klaus Westphal Dr.
rer. nat. Bernd Mussgnug Dr. rer. nat. Otto
Buchner Flossmannstrasse 30a
D-8000 München 60(DE)

(54) Gene coded for polypeptide which enhances virus infection of host insects.

(57) A gene coded for a polypeptide isolated from an occlusion body of a baculovirus such as Trichoplusia ni granulosis virus possessing a biological activity of enhancing virus infection of host insects by causing rapid degradation of the peritrophic membrane lining the midgut lumen of insects. Novel pesticides incorporating said gene or fragments thereof are also claimed.

EP 0 384 294 A2

# GENE CODED FOR A POLYPEPTIDE WHICH ENHANCES VIRUS INFECTION OF HOST INSECTS

The invention relates to the cloning and sequence of a novel virulence gene from a baculovirus for insect control. More particularly, the invention relates to the discovery of a DNA sequence and the deduced amino acid sequence for a polypeptide isolated from an occlusion body of a baculovirus and possessing a biological activity wherein said activity is. the enhancement of the infectivity of certain viral pesticides.

The publications used to illuminate the background of the invention, and in particular cases, to provide additional details respecting its practice are incorporated herein by reference, and for convenience, are numerically referenced by the following text and respectively grouped in the appended bibliography. Copies of all of the references mentioned in this bibliography are attached to the **INFORMATION DISCLOSURE STATEMENT**.

Present in the protein occlusion bodies (OBs) of some baculoviruses is a unique viral-encoded protein which enhances viral infection of the host insect. This protein has been referred to as the virus enhancing factor (VEF). Pest control compositions comprising this factor and nuclear polyhedrosis viruses are the subject matter of the copending application of one of the inventors, viz. Robert R. Granados, Serial No. 178,259, filed April 6, 1988 and entitled BACULOVIRUS PROTEINS AND VIRAL PESTICIDES CONTAINING SAME.

Studies on the mode of action of the VEF isolated from Trichoplusia ni granulosis virus (TnGV) showed that the VEF caused rapid degradation of the peritrophic membrane which lines the midgut lumen. Larval bioassays suggested that this alteration made the peritrophic membrane more permeable to invading baculoviruses resulting in at least a 25-fold increase in larval mortality (1, 2).

The closest prior art to the VEF protein of the present invention is believed to be a lipoprotein, originally isolated from a Hawaiian strain of Pseudaletia unipuncta granulosis virus, and described by Tanada and co-workers (7, 8, 10) as the "synergistic factor" (SF). While SF enhances nuclear polyhedrosis virus infection in larvae (7), the mode of action of the SF and the viral enhancing protein (VEP) appear to be different since the SF is an absorption factor, involved in the attachment of virions to cells in vivo and in vitro (10). In addition, phospholipase C which inactivates SF does not inactivate the VEP, and depolymerization did not occur even after a 4 hour incubation of the VEP with phospholipase C with and without 1% sodium dodecyl sulfate.

Since the viral enhancing protein(s) are important at early stages of host infection, it is important to identify and locate the position of the VEF gene on the viral genome. A need, therefore, exists to clone and sequence the VEF gene of Trichoplusia ni (cabbage looper) granulosis virus (TnGV) DNA (DNA is an abbreviation for deoxyribonucleic acid) and to show sequence homology of the VEF virulence gene among different baculoviruses. It is an object of this invention to satisfy such a need.

The above-mentioned object and other objects of the present invention which will hereinafter become more readily apparent from the following description have been attained by providing a VEF gene found in the granulin fraction of TnGV OBs purified by sephacryl column and comprising a DNA molecule encoding a polypeptide of molecular weight 101 kDa and possessing a biological activity wherein said activity is enhancement of infectivity of baculoviruses, and wherein said polypeptide has 900 amino acid residues in total in the amino acid sequence of the polypeptide.

A more complete appreciation of the invention and the attendant advantages thereof will be readily attained as the same becomes better understood by reference to the following details of description when considered in connection with the accompanying drawings, wherein:

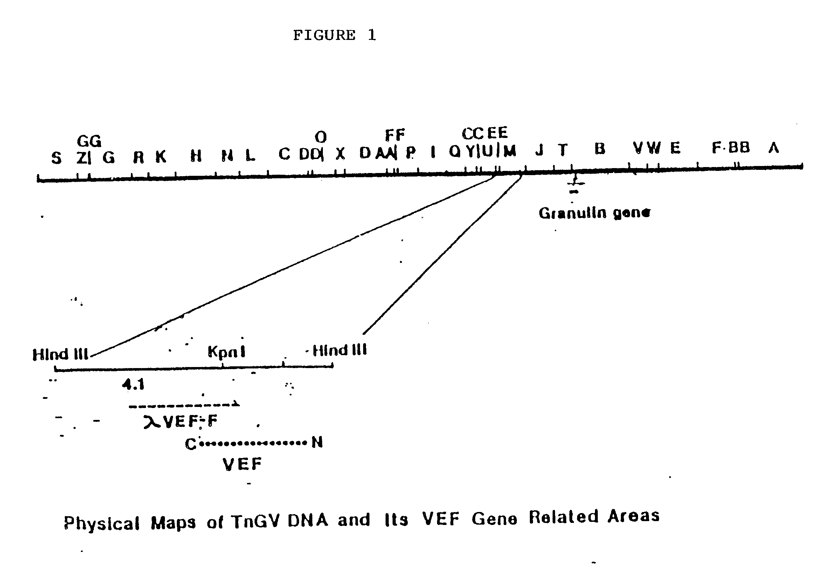

Fig. 1 is a summary of data showing the genome location of the inserts of positive recombinant phage and the gene location of the VEF.

Fig. 2a is a Hind III restriction map of TnGV DNA. By convention (4), the smallest fragment containing all of the granulin gene (bar and arrow) is assigned to be the first fragment of TnGV DNA. Asterisks indicate the position of shearing with a French press during the construction of a λ gt11 genomic library of TnGV DNA.

Fig. 2b is a fine map of the Hind III fragment of TnGV DNA. Since the VEF gene is located on the strand opposite to the one containing the granulin gene, the Hind III-M fragment is shown in the reverse orientation.

Figs. 2c and 2d show the structures of the fusion gene and the expressed fusion protein from the λ VEF-F clone. Solid lines in c and d show B-galactosidase gene and β-galactosidase in a fusion protein, respectively.

Fig. 2e shows predicted location of the VEF gene and sequencing strategy (arrows). Blocks with slash lines, closed blocks and open blocks show the predicted VEF gene, a carboxy terminal polypeptide expressed as a part of fusion proteins with β-galactosidase, and TnGV DNA flanking the VEF gene, respectively.

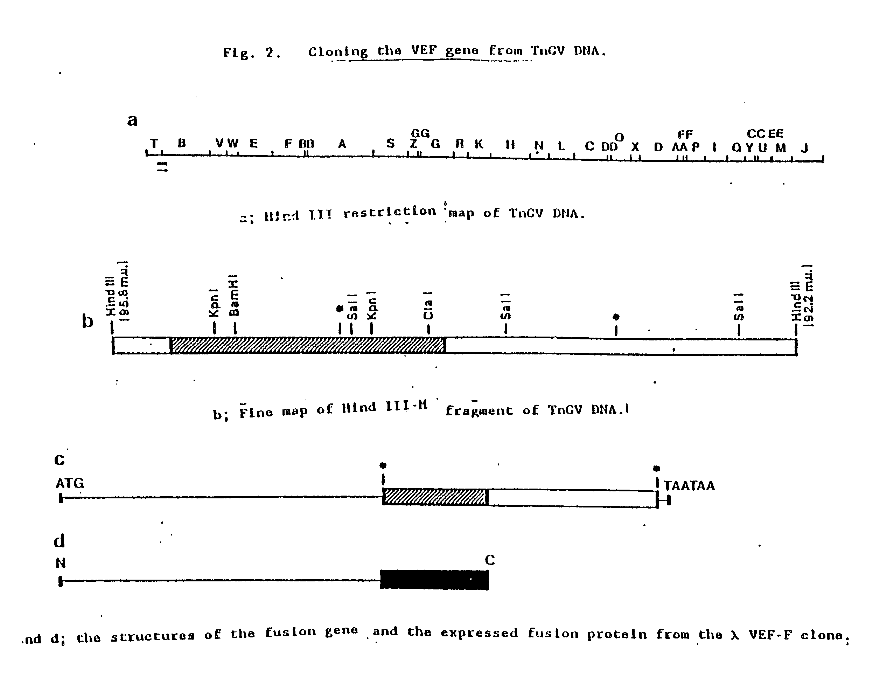

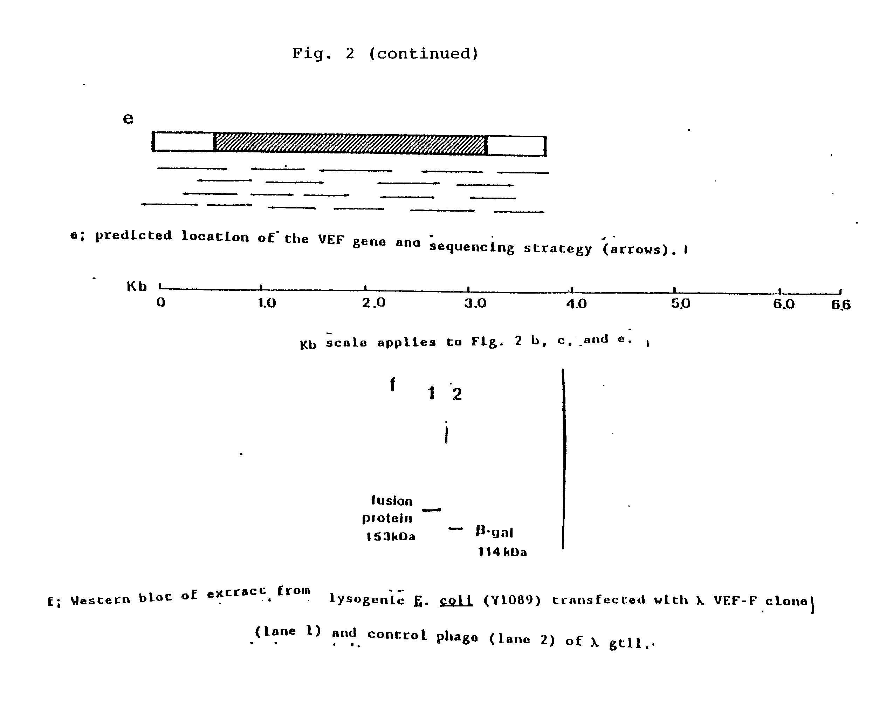

Kb scale applies to Figs. 2b, 2c, and 2e.

Fig. 2f is a Western blot of extract from lysogenic E. coli (Y1089) transfected with λ VEF-F clone (lane 1) and control phage (lane 2) of λ gt11. Visualization of the fusion protein and B-galactosidase was done by combining alkaline phosphatase-secondary antibody conjugates to either anti-VEF antiserum or anti-β galactosidase monoclonal antibody.

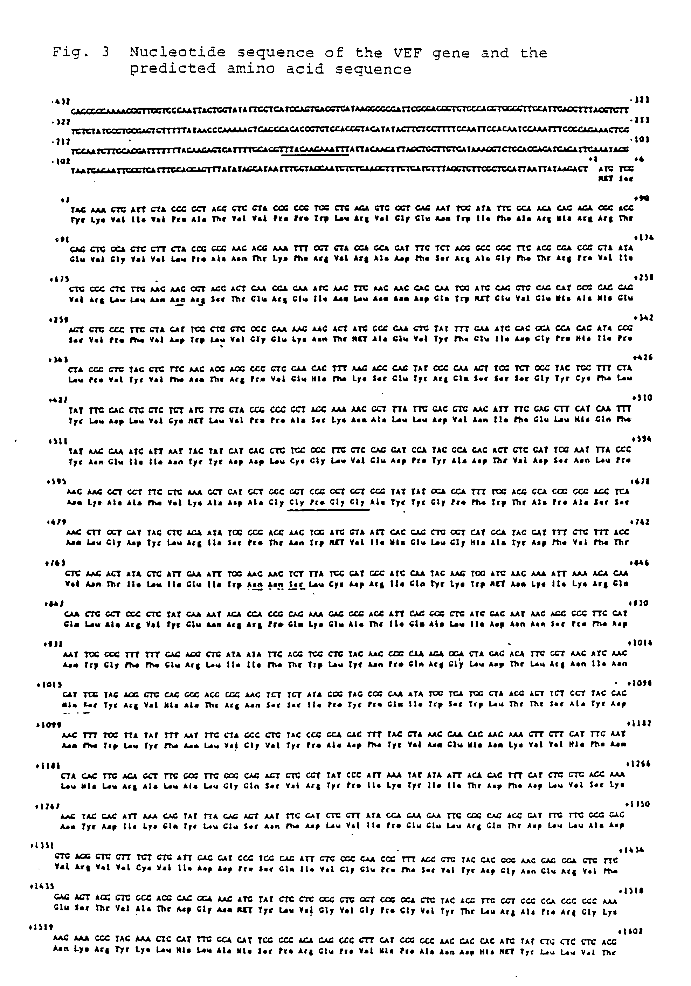

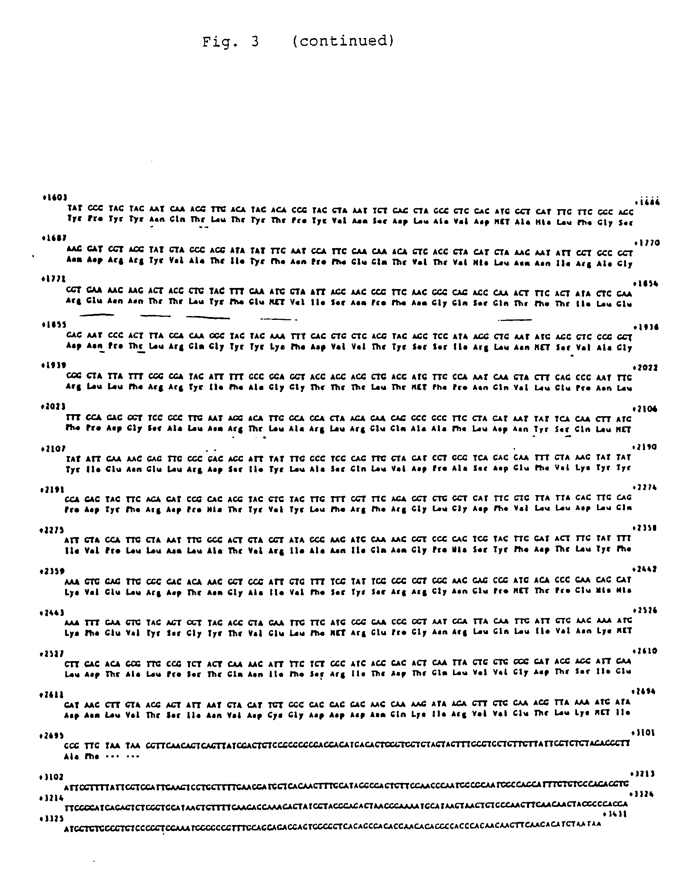

Fig. 3 shows the nucleotide sequence of the VEF gene and the predicted amino acid sequence. DNA sequence of a 3.5 Kb portion of Hind III-M fragment was determined by dideoxy chain termination method using bacteriophage T7 DNA polymerase. Sequence data were compiled and analyzed using the software program of PCGENE. A 14 mer consensus sequency (-169 to -155) commonly present in a transcription starting area in baculovirus genes expressed late in infection (5) and a sequence indicating a strong β-turn probability (+628 to +639) are also underlined. In this sequence, A stands for deoxyadenyl, G for deoxyguanyl, C deoxycytidyl, and T is thymidyl. The amino acids encoded by the above DNA are designated below the appropriate nucleotide triplet. Accordingly, MET is methionine; LYS is lysine; PRO is proline; GLU is glutamic acid; LEU is leucine; THR is threonine; ALA is alanine,; SER is serine; VAL is valine; PHE is phenylalanine; ILE is isoleucine; GLY is glycine; ASP is aspartic acid; GLN is glutamine; ARG is arginine; CYS is cysteine; TRP is tryptophan; ASN is asparagine; HIS is histidine; and TYR is tyrosine.

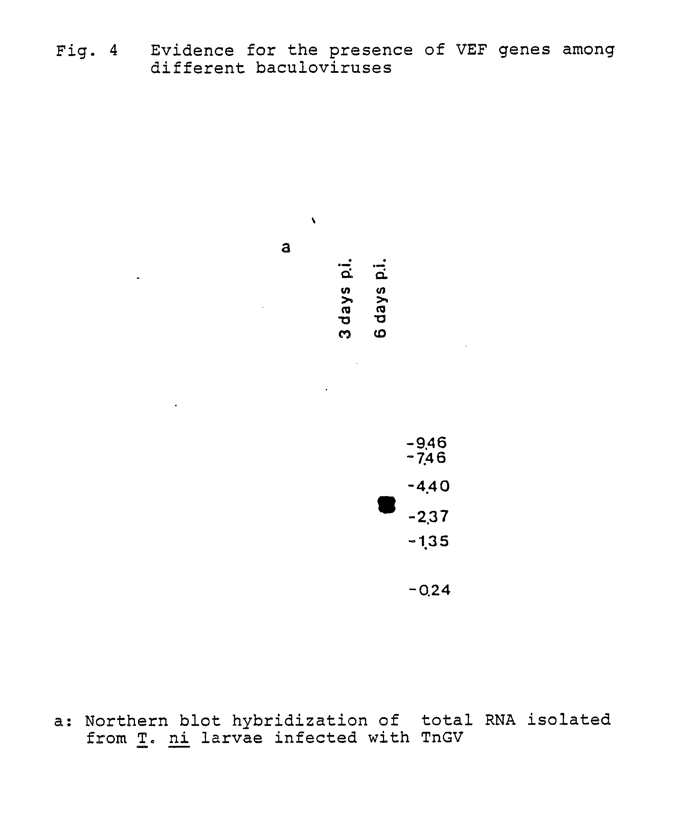

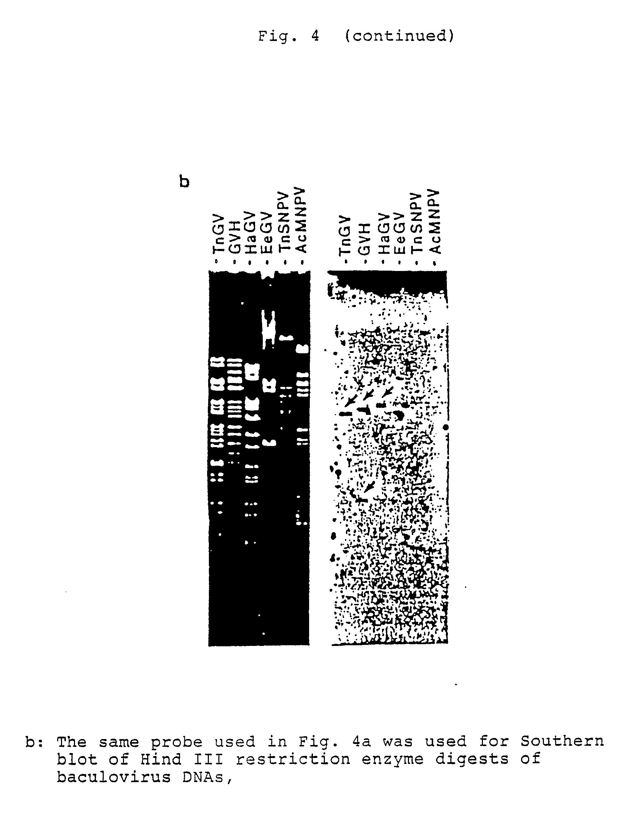

Fig. 4 shows evidence for the presence of VEF genes among different baculoviruses.

Fig. 4a is Northern blot hybridization of total RNA isolated from T. ni larvae infected with TnGV at 3 and 6 days post inoculation (p.i.) with a probe consisting of a TnGV KpnI fragment (95.2 m.u. to 94.4 m.u., Fig. 2b).

Fig. 4b shows that the same probe used in Fig. 4a was used for Southern blot of Hind III restriction enzyme digests of baculovirus DNAs (TnGV; Heliothis armigera GV, HaGV; GVH; T. ni SNPV, TnSNPV; Erinnyis ello GV, EeGV; A. californica MNPV, AcMNPV). Left panel shows Hind III restriction fragments on a 0.7% agarose gel. Right panel shows autoradiogram of Southern blot and DNA fragment with sequences homologous to the probe (arrows).

Fig. 4c is an SDS-polyacrylamide gel electrophoresis analysis of granulin and polyhedrin fractions from baculoviruses. The OBs from baculoviruses listed in the above paragraph were solubilized in 50 mM sodium carbonate for 10 min at room temperature. The virus particles and the unsolubilized OBs were pelleted through a 20% sucrose cushion by centrifugation at 126,000 g for 30 min. The upper phase containing the granulin or polyhedrin fraction was incubated at 28°C overnight to degrade components sensitive to endogenous alkaline protease derived from the host midgut (15). The samples were run on 12.5% SDS-polyacrylamide gel and stained with silver (1). The TnGV VEF is a polypeptide of molecular weight of 101 kDa (asterisk).

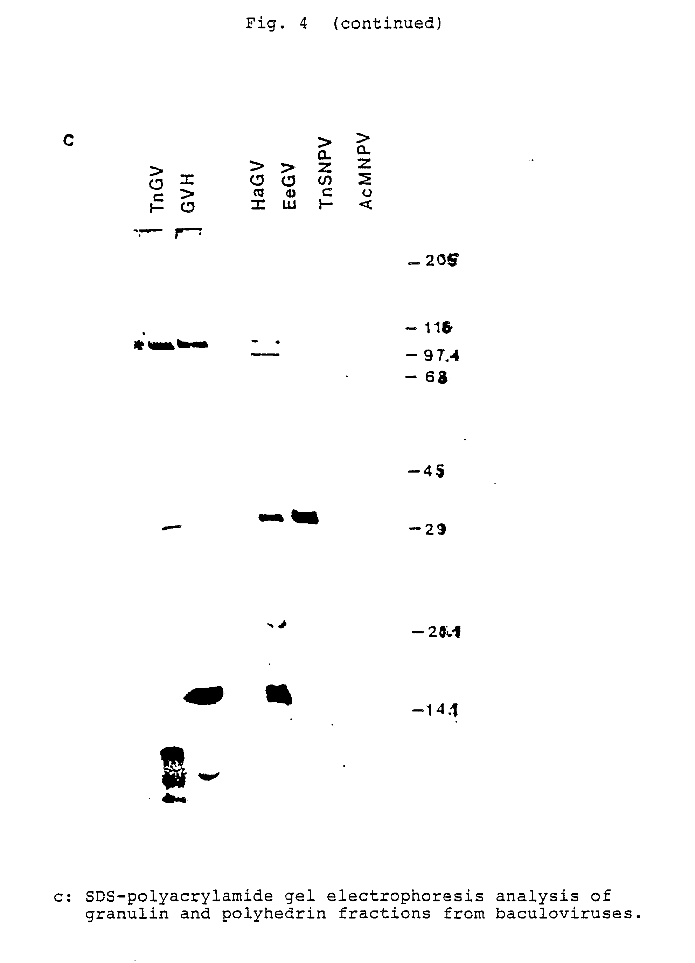

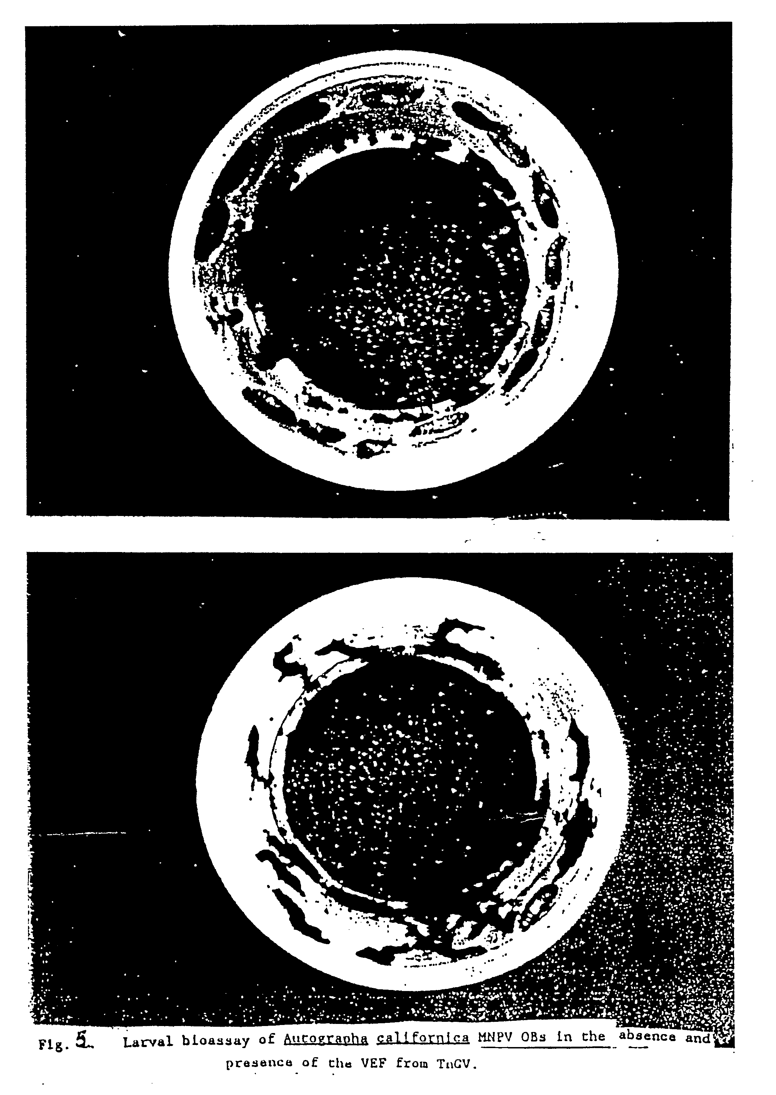

Fig. 5 is a larval bioassay of Autographa californica MNPV OBs in the absence and presence of the VEF from TnGV. Newly molted 5th instar T. ni larvae were inoculated per os with 2 μl of Autographa californica MNPV OBs at a concentration (1 OB/larva) adjusted to obtain 17% mortality of treated insects (a). A second group of larvae were treated with the same OB concentration along with 40 ng of the purified VEF protein (b). When the VEF was present in the inoculum (a), 95% mortality occurs.

The VEF present in the granulin fraction of TnGV OBs was purified in the following manner:

$1.7 \times 10^{-12}$ TnGV OBs were dissolved in 1 ml 0.05 M $Na_2CO_3$ for 15 min. at room temperature, and layered on a 20% sucrose cushion in $H_2O$ and centrifuged for 45 min. at 126,000 g at 4°C. The granulin fraction remained on top of the sucrose cushion and was collected. After an incubation of 5 hrs at 28°C, the granulin fraction was applied onto a Sephacryl-S-200 column (2.6 x 34 cm) and eluted with 50 mM Tris-HCl pH 7.0, 0.1 M NaCl at 1.5 ml/min, and the absorption of the eluate measured at 280 nm. The first peak containing VEF protein was pooled and used for experiment. D, dead larvae; P, healthy pupa.

Following purification by means of the sephacryl column, the biological activity of the protein was determined by larval bioassay (Fig. 5). As little as 40 ng of VEF, in addition to the virus inoculum, per larva was sufficient to increase the infection of fifth instar T. ni larvae from 17% to 95% (see Fig. 5).

A cloning and expression vector, λ gt11, was used for construction of a genomic library of TnGV and for isolation of the VEF gene (3). Antibodies were raised against Sephacryl-column purified VEF from granulin fraction after alkali solubilization of OBs (1) and were used for immunoblotting to screen for positive clones. Through several steps of screening approximately 6000 plaques, a clone was selected containing the longest viral DNA insert, λ VEF-F (Fig. 2). Southern blot hybridization analysis of TnGV DNA Hind III digests, probed with the λ VEF-F clone insert, revealed that the VEF gene existed on the Hind III-M fragment. Western blot analysis of the fusion protein expressed in lysogenic E. coli (Y1089 strain) transfected with λ VEF-F had a molecular weight of 153 kDa. This suggested a fusion protein gene consisting of 39kDa of the VEF carboxy terminal end and the 114 kDa beta-gal gene. Since the VEF has a size of 101 kDa (1), the position of the VEF gene on a fine map of the Hind III-M fragment was predicted and a 3.5 kbp DNA portion was sequenced (Fig. 2).

Sequence analysis showed an open reading frame of 2.7 kbp DNA corresponding to the size of the VEF polypeptide at the predicted location of the VEF gene (Fig. 3). The deduced size of the polypeptide was 101,200 daltons and consisted of 900 amino acid residues. There are no sites for lipophilic modification (Lys/X/X/Cys/X/X/Asn). The gene has a 14-mer consensus sequence transcriptional signal known as the Rohrmann Box and found in other baculovirus genes which are expressed late in the infection cycle (5).

To determine the presence of the VEF gene among several isolates of baculoviruses a 1.5 kbp portion of the VEF gene was probed onto a Southern blot of different virus DNA fragments digested with Hind III restriction enzyme under high stringency condition (12). The result showed that two granulosis virus DNAs, isolated from P. unipuncta-Hawaiian strain (GVH) and Heliothis armigera - (HaGV), contained a sequence homologous to the TnGV VEF gene (Fig. 4a). DNA isolated from Erinnyis ello (EeGV) did not contain sequences homologous to the VEF gene probe. The restriction enzyme digestion pattern of DNA from TnGV, GVH, and HaGV were very similar, whereas EeGV exhibited a very distinct DNA Profile. The probe did not hydridize with DNAs from two nuclear polyhedrosis viruses (Fig. 4a). Temporal gene expression of the VEF gene was examined by Northern blot analysis of total RNA from TnGV-infected T. ni larvae at 3 and 6 days p.i. A probe with a size of 1.5 Kb kpnl-V fragment, a part of the VEF gene, showed no hybridization with RNAs at 3 days p.i. but showed strong hybridization with two RNA species with sizes of 2.7 Kb and 3.3 Kb at 6 days p.i. (Fig. 4b). The TnGV-VEF present in the granulin fraction of alkaline dissolved OBs was resolved as a 101 kDa protein on a SDS-polyacrylamide gel. To determine the presence of high molecular weight polypeptides in granulin or polyhedrin fractions from six baculoviruses, these virus samples were analyzed by SDS-PAGE (Fig. 4c). In the granulin fractions from TnGV, GVH and HaGV, polypeptides with a size of 101 KDa, 103 kDa, and a complex of 104 kDa and 94 kDa were detected, respectively. The single high molecular weight polypeptide (103 kDa) from GVH appears to be analogous to the 101 kDa protein from TnGV (Fig. 4c and Y. Tanada, personal communication). The assignment of a VEF function to either the 94 or 104 kDa polypeptides from HaGV is not clear at this time. No polypeptides with a size of approximately 100 kDa were present in EeGV, TnSNPV, and ACMNPV. Three of the GVs examined, TnGV, GVH, and HaGV all infect the noctuid species T. ni, whereas EeGV grows only in the sphyngid species, E. ello.

The coded gene of the present invention can be used in engineering new viral pesticides with enhanced efficacy. For example, it can be used in combination with known biological insecticides such as BT or with synthetic chemical insecticides. The gene product of this invention can also be used to produce VEF in any microbial production system, e.g. E. coli., bacillus or streptococcus. It can be introduced into a variety of hosts such as plants for protection against insects or microbes as biological active agents.

The coded gene of the present invention has been found to play a significant role as a determinant of virulence at the initial stage of infection in insect hosts. Knowledge gained in cloning and sequencing the VEF gene should prove useful in helping to unravel the mechanism(s) of enhanced virus infection by enhancement factors present within the occlusion body matrix.

Notwithstanding that reference has been made to particular preferred embodiments, it will be understood that the present invention is not to be construed as limited as such, but rather to the lawful scope of the appended claims. In other words, the subject invention includes not only the specific nucleotide sequences depicted herein, but also all equivalent nucleotide sequences coding for molecules with substantially the same biological activity of enhancing the infectivity of baculoviruses. The term "equivalent" is being used in ordinary patent usage here as denoting a nucleotide sequence which performs substantially as the nucleotide sequence identified herein to produce molecules with substantially the same biological activity in essentially the same kind of hosts. Within this definition are subfragments which have biological activity of enhancing the infectivity of baculoviruses.

Inasmuch as the protein, i.e., the gene product, of the present invention has been defined by means of deductive amino acid sequencing, c.f. Fig. 3, it is to be understood that for this particular protein, embraced herein, natural allelic variations exist and occur from individual to individual. These variations may be demonstrated by (an) amino acid difference(s) in the overall sequence or by deletions, substitutions, insertions, inversions or additions of (an) amino acid(s) in said sequence. All such alletic variations are included within the scope of the present invention.

## BIBLIOGRAPHY

1. Derksen, A.G.S. and Granados, R.R. Virology. 167: 242-250 (1981).

2. Peters, W. and Wiese, B. J. Insect Physio. 32:43-49 (1986).

3. Snyder, M., Sweatser, D., Young, R.A. and Davis, R.W. Meth. Enzym. 154:107-128 (1985).

4. Vlak, J.M. and Smith, G.E. J. Virol. 41:1118-1121 (1981).

5. Rohrmann, G.F. J. Gen. Virol. 67:1499-1513 (1986).

6. Tanada, V. and Hukuhara, T. J. Invertebr. Pathol. 17:116-126 (1971).

7. Tanada, Y. and Hara, S. Nature. 254:328-329 (1975).

8. Tanada, Y. J. Insect Pathol. 1:215-231 (1959).

9. Tanada, Y. Inoue, H., Hess, R.T., and Omi, E.M., J. Inverebr. Pathol. 34:249-255 (1980).

10. Tanada, Y. J. Invertebr. Pathol. 45:125-138 (1985).

11. Yamamoto, T., Kita, H. and Tanada, Y. J. Gen. Virol. 45:371-381 (1979).

12. Maniatis, T., Fritsch, E.F. and Sambrook, J. Molecular Cloning: A Laboratory Manual. Cold Spring Harbor Laboratory, Cold Spring Harbor, N.Y. (1982).

13. Hamm, J.J. and Paschke, J.D. J. Insect Pathol. 5:187-197 (1963).

14. Whitlock, V.H. J. Invertebr. Pathol. 23:70-75 (1974).

15. Granados, R.R. and Williams, K.A. The Biology of Baculoviruses, CRC Press, Fl. Vol I:89-108 (1986).

16. Miller, L.K. The Biology of Baculoviruses, CRC Press, Fl. Vol I:217-238 (1986).

## Claims

1. A gene comprising a DNA molecule encoding a polypeptide possessing a biological activity wherein said activity is the enhancement of infectivity of baculoviruses wherein said polypeptide has about 900 amino acid residues in total in the amino acid sequence of the polypeptide.

2. The gene product of Claim 1 whose size is 101,200 daltons.

3. The gene product of Claim 1 isolated from the protein occlusion bodies of Trichoplusia ni granulosis virus.

4. The gene of Claim 1 which encodes a polypeptide having the amino acid sequence of Fig. 3.

5. The gene of Claim 4 having a 14-mer consensus transcriptional signal.

6. A method for improving the efficacy of viral pesticides which comprises treating the pest with a viral composition containing the VEF gene of Claim 1.

7. A viral insecticide comprising a polypeptide characterized by having the amino acid sequence of Fig. 3.

8. A composition comprising the gene product of Claim 1 and a pesticide.

9. A toxicant composition comprising the gene product of Claim 1 and a biological insecticide.

10. A toxicant composition comprising the gene product of Claim 1 and a synthetic chemical insecticide.

11. A microbial or cellular protein system for the production of the viral enhancing factor (VEF) which includes the gene product of Claim 1.

12. The microbial system of Claim 11 in which the microorganisms are selected from the group consisting of E. coli, bacillus, and streptococcus.

13. Process of introducing the gene product of Claim 1 into plants for protection against insects.

14. Process of introducing the gene product of Claim 1 into microbes useful as biologically active agents.

15. A gene comprising a DNA molecule encoding a polypeptide possessing a biological activity wherein said activity is the enhancement of infectivity of baculoviruses and wherein the DNA sequence differs from that exhibited in the gene of Claim 1 only in its amino acid composition at non-critical positions.

FIGURE 1

Physical Maps of TnGV DNA and Its VEF Gene Related Areas

EP 0 384 294 A2

Fig. 2.    Cloning the VEF gene from TnGV DNA.

a

T   B    VW  E    F BB   A      S GG G  R  K   II   N  L   C DD O  X  D FF P  I  CC EE J
                              Z                        DD    AA      Q Y U M

a; Hind III restriction map of TnGV DNA.

b

Hind III    Kpn I   Sal I  Cla I    Sal I        Sal I   Hind III
(95.8 m.u.)  BamHI  Kpn I                                (92.2 m.u.)

b; Fine map of Hind III-M fragment of TnGV DNA.

c

ATG                                          TAATAA

d

N                                    C

.nd d; the structures of the fusion gene and the expressed fusion protein from the λ VEF-F clone.

EP 0 384 294 A2

# Fig. 2 (continued)

**e**

e; predicted location of the VEF gene and sequencing strategy (arrows).

Kb scale: 0   1.0   2.0   3.0   4.0   5.0   6.0   6.6

Kb scale applies to Fig. 2 b, c, and e.

**f**   1   2

fusion
protein —
153kDa   — β-gal
114 kDa

f; Western blot of extract from lysogenic E. coli (Y1089) transfected with λ VEF-F clone (lane 1) and control phage (lane 2) of λ gt11.

EP 0 384 294 A2

Fig. 3  Nucleotide sequence of the VEF gene and the
predicted amino acid sequence

CACCCCAAAACCGTTCGTCCCAATTACTCGTATATTCGTCATCCAGTCACGTCATAAACCCGCCATTCGCCACCGTCTCCCACGTGCCCGTTCCATTCACGTTTACGTCTT

TCTGTATCCGTCCCAGTCGTTTTTATAACCCAAAAACTCAGCCACACCGTCTCCACCGTACATATACTTCTCCGTTTTCCAATTCCACAATCCAAATTTCCCCACAAACTCC

TCCAATCTTCCACCCATTTTTTTACAACAGTCATTTTGCACGTTTACAACAAAATTTATTACAACGATTACGTCGTTCGTCATAAAACGTCTCCACCACATCACATTCAAATACG

TAATCACAACTTCGGTCATTTCCACCCAGTTTATATACCCATAATTTCGTACCCAATCTCTCAAACGTTTCGTCATGTTTACGTCTTCCGCTCCATTAATTATAACGACT   ATG TCC
                                                                                                                 MET Ser

TAC AAA GTC ATT CTA CCC CCT ACC GTC CTA CCG CCG TCG CTC ACA CTC CGT CAG AAT TCG ATA TTC CCA ACA CAC ACA CCC ACC
Tyr Lys Val Ile Val Pro Ala Thr Val Val Pro Pro Trp Leu Arg Val Gly Glu Asn Trp Ile Phe Ala Arg His Arg Arg Thr

GAG CTC GCA GTC CTT CTA CCG CCG AAC ACG AAA TTT CGT GTA CCA GCA GAT TTC TCT ACG CCC CCC TTC ACG CCA CCC CTA ATA
Glu Val Gly Val Val Leu Pro Ala Asn Thr Lys Phe Arg Val Arg Ala Asp Phe Ser Arg Ala Gly Phe Thr Arg Pro Val Ile

GTC CCC CTC TTG AAC AAC CGT ACC ACT CAA CCA CAA ATC AAC TTC AAC AAC CAC CAA TCG ATC CAG GTC CAG CAT CCC CAC CAG
Val Arg Leu Leu Asn Asn Arg Ser Thr Glu Arg Glu Ile Asn Leu Asn Asn Asp Gln Trp MET Glu Val Glu His Ala His Glu

ACT GTC CCC TTC CTA GAT TCG CTC GTC CCC CAA AAG AAC ACT ATC CCC CAA GTG TAT TTT GAA ATC CAC CCA CCA CAC ATA CCC
Ser Val Pro Phe Val Asp Trp Leu Val Gly Glu Lys Asn Thr MET Ala Glu Val Tyr Phe Glu Ile Asp Gly Pro His Ile Pro

CTA CCC CTC TAC CTC TTC AAC ACC ACG CCC CTC CAA CAC TTT AAG ACC CAG TAT CCC CAA ACT TCC TCT CCC TAC TCC TTT CTA
Leu Pro Val Tyr Val Phe Asn Thr Arg Pro Val Glu His Phe Lys Ser Glu Tyr Arg Gln Ser Ser Ser Gly Tyr Cys Phe Leu

TAT TTC CAC CTC CTC TCT ATC TTC CTA CCG CCC CCT ACC AAA AAC GCT TTA TTC CAC CTC AAC ATT TTC CAG CTT CAT CAA TTT
Tyr Leu Asp Leu Val Cys MET Leu Val Pro Pro Ala Ser Lys Asn Ala Leu Leu Asp Val Asn Ile Phe Glu Leu His Gln Phe

TAT AAC CAA ATC ATT AAT TAC TAT CAT CAC CTC TCC CCC TTG CTC CAG CAT CCA TAC CCA CAC ACT GTC CAT TCG AAT TTA CCC
Tyr Asn Glu Ile Ile Asn Tyr Tyr Asp Asp Leu Cys Gly Leu Val Glu Asp Pro Tyr Ala Asp Thr Val Asp Ser Asn Leu Pro

AAC AAG CCT CCT TTC CTG AAA CCT CAT CCT CCC CCT CCC CCT CCT CCG TAT TAT CCA CCA TTT TCG ACG CCA CCG CCC ACC TCA
Asn Lys Ala Ala Phe Val Lys Ala Asp Ala Gly Gly Pro Gly Gly Ala Tyr Tyr Gly Pro Phe Trp Thr Ala Pro Ala Ser Ser

AAC CTT CGT CAT TAC CTC ACA ATA TCG CCC ACC AAC TCG ATC GTA ATT CAC CAG CTC CGT CAT CCA TAC CAT TTT CTC TTT ACC
Asn Leu Gly Asp Tyr Leu Arg Ile Ser Pro Thr Asn Trp MET Val Ile His Glu Leu Gly His Ala Tyr Asp Phe Val Phe Thr

GTC AAC ACT ATA CTC ATT CAA ATT TCG AAC AAC TCT TTA TCG CAT CCC ATC CAA TAC AAG TCG ATC AAC AAA ATT AAA ACA CAA
Val Asn Thr Ile Leu Ile Glu Ile Trp Asn Asn Ser Leu Cys Asp Arg Ile Gln Tyr Lys Trp MET Asn Lys Ile Lys Arg Gln

CAA CTC CCT CCC CTC TAT CAA AAT ACA CCA CCG CAG AAA CAG CCG ACC ATT CAG GCG CTC ATC CAC AAT AAC ACC CCG TTC CAT
Gln Leu Ala Arg Val Tyr Glu Asn Thr Arg Pro Gln Lys Glu Ala Thr Ile Gln Ala Leu Ile Asp Asn Asn Ser Pro Phe Asp

AAT TCG CCC TTT TTT CAG ACG CTC ATA ATA TTC ACG TCG CTC TAC AAC CCC CAA ACA CCA CTA CAC ACA TTC CGT AAC ATC AAC
Asn Trp Gly Phe Phe Glu Arg Leu Ile Ile Phe Thr Trp Leu Tyr Asn Pro Gln Arg Gly Leu Asp Thr Leu Arg Asn Ile Asn

CAT TCG TAC ACG GTC CAC CCC ACC CGC AAC TCT TCT ATA CCG TAG CCG CAA ATA TCG TCA TCG CTA ACG ACT TCT CCT TAC CAC
His Ser Tyr Arg Val His Ala Thr Arg Asn Ser Ser Ile Pro Tyr Pro Gln Ile Trp Ser Trp Leu Thr Thr Ser Ala Tyr Asp

AAC TTT TCG TTA TAT TTT AAT TTG CTA GCC CTG TAC CCG CCA CAC TTT TAC GTA AAC CAA CAC AAC AAA GTT GTT CAT TTC AAT
Asn Phe Trp Leu Tyr Phe Asn Leu Val Gly Val Tyr Pro Ala Asp Phe Tyr Val Asn Glu His Asn Lys Val Val His Phe Asn

CTA CAC TTG ACA GCT TTC CCG TTC CCG CAG ACT CTC CGT TAT CCC ATT AAA TAT ATA ATT ACA CAC TTT CAT CTC CTC ACC AAA
Leu His Leu Arg Ala Leu Ala Leu Gly Gln Ser Val Arg Tyr Pro Ile Lys Tyr Ile Ile Thr Asp Phe Asp Leu Val Ser Lys

AAC TAC CAC ATT AAA CAG TAT TTA CAG ACT AAT TTC CAT CTC GTT ATA CCA CAA CAA TTG CCG CAC ACC CAT TTG TTC CCG CAC
Asn Tyr Asp Ile Lys Gln Tyr Leu Glu Ser Asn Phe Asp Leu Val Ile Pro Glu Glu Leu Arg Gln Thr Asp Leu Leu Ala Asp

CTC ACG GTC GTT TCT CTC ATT CAC GAT CCG TCG CAG ATT CTC CCC CAA CCC TTT ACC CTC TAC CAC CCG AAC CAG CCA CTC TTC
Val Arg Val Val Cys Val Ile Asp Asp Pro Ser Gln Ile Val Gly Glu Pro Phe Ser Val Tyr Asp Gly Asn Glu Arg Val Phe

CAG ACT ACG CTC CCC ACC CAC CCA AAC ATC TAT CTC CTC CCC CTC CGT CCC CCA CTC TAC ACC TTC CGT CCG CCA CCG CCC AAA
Glu Ser Thr Val Ala Thr Asp Gly Asn MET Tyr Leu Val Gly Val Gly Pro Gly Val Tyr Thr Leu Arg Ala Pro Arg Gly Lys

AAC AAA CCC TAC AAA CTC CAT TTG CCA CAT TCG CCC ACA CAG CCC CTT CAT CCG CCC AAC CAC CAC ATC TAT CTC CTC CTG ACG
Asn Lys Arg Tyr Lys Leu His Leu Ala His Ser Pro Arg Glu Pro Val His Pro Ala Asn Asp His MET Tyr Leu Leu Val Thr

Fig. 3 (continued)

+1603                                                                                                                    +1686
TAT CCC TAC TAC AAT CAA ACG TTG ACA TAC ACA CCG TAC CTA AAT TCT CAC CTA GCC GTC GAC ATG GCT CAT TTG TTC CGC ACC
Tyr Pro Tyr Tyr Asn Gln Thr Leu Thr Tyr Thr Pro Tyr Val Asn Ser Asp Leu Ala Val Asp MET Ala His Leu Phe Gly Ser

+1687                                                                                                                    +1770
AAC GAT CGT ACG TAT GTA CCC ACG ATA TAT TTC AAT CCA TTC GAA CAA ACA GTC ACC GTA CAT CTA AAC AAT ATT CGT GCC GGT
Asn Asp Arg Arg Tyr Val Ala Thr Ile Tyr Phe Asn Pro Phe Glu Gln Thr Val Thr Val His Leu Asn Asn Ile Arg Ala Gly

+1771                                                                                                                    +1854
CGT GAA AAC AAG ACT ACC CTC TAC TTT CAA ATG GTA ATT ACC AAC CCG TTC AAC GGC CAG ACC CAA ACT TTC ACT ATA CTC CAA
Arg Glu Asn Asn Thr Thr Leu Tyr Phe Glu MET Val Ile Ser Asn Pro Phe Asn Gly Gln Ser Gln Thr Phe Thr Ile Leu Glu

+1855                                                                                                                    +1938
CAC AAT CCC ACT TTA CCA CAA GCC TAC TAC AAA TTT CAC GTC GTC ACG TAC AGC TCC ATA ACG CTG AAT ATG AGC GTC GCG GGT
Asp Asn Pro Thr Leu Arg Gln Gly Tyr Tyr Lys Phe Asp Val Val Thr Tyr Ser Ser Ile Arg Leu Asn MET Ser Val Ala Gly

+1939                                                                                                                    +2022
CGG CTA TTA TTT CGG CGA TAC ATT TTT GCC GGA GGT ACC ACC ACG CTG ACC ATG TTC CCA AAT CAA GTA CTT GAG CCC AAT TTG
Arg Leu Leu Phe Arg Arg Tyr Ile Phe Ala Gly Gly Thr Thr Thr Leu Thr MET Phe Pro Asn Gln Val Leu Glu Pro Asn Leu

+2023                                                                                                                    +2106
TTT CCA GAC GGT TCC GCC TTG AAT ACG ACA TTG CCA CCA CTA ACA CAA CAG CCC GCC TTC CTA CAT AAT TAT TCA CAA CTT ATC
Phe Pro Asp Gly Ser Ala Leu Asn Arg Thr Leu Ala Arg Leu Arg Glu Gln Ala Ala Phe Leu Asp Asn Tyr Ser Gln Leu MET

+2107                                                                                                                    +2190
TAT ATT CAA AAC CAG TTG CGC CAC ACC ATT TAT TTG CCC TCC CAG TTG CTA CAT CCT GCG TCA CAC CAA TTT CTA AAC TAT TAT
Tyr Ile Gln Asn Gln Leu Arg Asp Ser Ile Tyr Leu Ala Ser Gln Leu Val Asp Pro Ala Ser Asp Glu Phe Val Lys Tyr Tyr

+2191                                                                                                                    +2274
CCA GAC TAC TTC ACA GAT CCG CAC ACG TAC GTC TAC TTG TTT CGT TTC ACA GCT CTC GCT CAT TTC GTG TTA TTA GAC TTG CAG
Pro Asp Tyr Phe Arg Asp Pro His Thr Tyr Val Tyr Leu Phe Arg Phe Arg Gly Leu Gly Asp Phe Val Leu Leu Asp Leu Gln

+2275                                                                                                                    +2358
ATT GTA CCA TTG CTA AAT TTG GCC ACT GTA CGT ATA GCC AAG ATC CAA AAC CGT CCC CAC TCG TAC TTC GAT ACT TTG TAT TTT
Ile Val Pro Leu Leu Asn Leu Ala Thr Val Arg Ile Ala Asn Ile Gln Asn Gly Pro His Ser Tyr Phe Asp Thr Leu Tyr Phe

+2359                                                                                                                    +2442
AAA GTG GAG TTG CGC GAC ACA AAC GGT GCG ATT GTG TTT TCG TAT TCG CGC CGT CGC AAC GAG CCG ATG ACA CCC GAA CAC CAT
Lys Val Glu Leu Arg Asp Thr Asn Gly Ala Ile Val Phe Ser Tyr Ser Arg Arg Gly Asn Glu Pro MET Thr Pro Glu His His

+2443                                                                                                                    +2526
AAA TTT GAA GTG TAC AGT GGT TAC ACC GTA GAA TTG TTC ATG CGG GAA CCC GGT AAT CCA TTA CAA TTG ATT GTC AAC AAA ATC
Lys Phe Glu Val Tyr Ser Gly Tyr Thr Val Glu Leu Phe MET Arg Glu Pro Gly Asn Arg Leu Gln Leu Ile Val Asn Lys MET

+2527                                                                                                                    +2610
CTT GAC ACA GCG TTG CCG TCT ACT CAA AAC ATT TTC TCT CGC ATC ACC GAC ACT CAA TTA GTG CTC GCG GAT ACG ACG ATT CAA
Leu Asp Thr Ala Leu Pro Ser Thr Gln Asn Ile Phe Ser Arg Ile Thr Asp Thr Gln Leu Val Val Gly Asp Thr Ser Ile Glu

+2611                                                                                                                    +2694
GAT AAC CTT GTA ACG ACT ATT AAT GTA GAT TGT GGC GAC GAC GAC AAC CAA AAG ATA ACA CTT GTC GAA ACG TTA AAA ATC ATA
Asp Asn Leu Val Thr Ser Ile Asn Val Asp Cys Gly Asp Asp Asp Asn Gln Lys Ile Arg Val Val Glu Thr Leu Lys MET Ile

+2695                                                                                                                    +3101
GCG TTC TAA TAA CGTTCAACAGTCAGTTATCGACTGTCCGCGCGCCGACCACATGACACTGCGTCGTGTAGTACTTTGCGTGCTGTTGTTATTCGTCTGTACACCGTT
Ala Phe ... ...

+3102                                                                                                                    +3213
ATTCGTTTTATTCGTCCATTCAAGTCCTGCTTTTCAACCATGCTCACAACTTTGCATAGCCGACTGTTCCAACCCAATCGCCCAATGCCCACCATTTCTGTCTCCACACGTC
+3214                                                                                                                    +3324
TTCCGGCATCAGAGTCTCGGTGCCATAACTGTTTTCAACACCAAACACTATCGTACCGCACACTAACCCAAAATGCCATAACTAACTGTGCCCAACTTCAACAACTACGCCCACCA
+3325                                                                                                                    +3411
ATGCTGTGCCCGTGTCCCCGTCCAAATGCCGCCCCGTTTGCAGGCAGACCACTGCCCGCTCACAGCCCACACCAACACACCCCACCCACAACAAGTTCAACACATCTAATAA

Fig. 4    Evidence for the presence of VEF genes among
          different baculoviruses

a

3 days p.i.

6 days p.i.

-9,46
-7,46

-4,40

-2,37

-1,35

-0,24

a: Northern blot hybridization of  total RNA isolated
   from T. ni larvae infected with TnGV

EP 0 384 294 A2

Fig. 4  (continued)

b: The same probe used in Fig. 4a was used for Southern
   blot of Hind III restriction enzyme digests of
   baculovirus DNAs,

Fig. 4 (continued)

c: SDS-polyacrylamide gel electrophoresis analysis of
   granulin and polyhedrin fractions from baculoviruses.

Fig. 5. Larval bioassay of _Autographa californica_ MNPV OBs in the absence and presence of the VEF from TnGV.